# EUROPEAN PATENT SPECIFICATION

(11) **EP 3 813 928 B1**
(45) Date of publication and mention of the grant of the patent: **09.08.2023**
(21) Application number: 19826733.8
(22) Date of filing: 26.06.2019
(51) Int. Cl.: A61N 1/36, A61N 1/375

(54) **IMPLANTABLE NEURAL STIMULATION DEVICE WITH TWO HEADERS**
IMPLANTIERBARE NERVENSTIMULATIONSVORRICHTUNG MIT ZWEI KOPFTEILEN
DISPOSITIF DE STIMULATION NEURONALE IMPLANTABLE À DEUX EMBOUTS

(30) Priority: 29.06.2018 AU 2018902346; 18.04.2019 AU 2019901356
(43) Date of publication of application: 05.05.2021
(73) Proprietor: Saluda Medical Pty Ltd, Artarmon, New South Wales 2064 (AU)
(72) Inventor: SINGLE, Peter Scott Vallack, Artarmon, New South Wales 2064 (AU); PARKER, John Louis, Artarmon, New South Wales 2064 (AU)
(74) Representative: Abel & Imray LLP
(86) International application number: PCT/AU2019/050665
(87) International publication number: WO 2020/000039

(56) References cited:
- WO-A1-2004/087256
- US-A- 5 313 953
- US-A- 6 026 328
- US-A1- 2010 019 985
- US-A1- 2011 190 833
- US-A1- 2011 190 842
- US-A1- 2014 350 635
- US-A1- 2017 100 597
- US-A1- 2017 303 424
- US-A1- 2019 060 656
- US-B2- 8 929 986

## Description

### Technical Field

The present disclosure relates to an implantable neural stimulation device. The device may, in some examples, be used to treat medical conditions.

### Background

Medical devices having one or more active implantable components have provided a wide range of therapeutic benefits to patients over recent decades. Implantable neural stimulation devices are an example of such medical devices and are used to generate and deliver electrical pulses to tissue to treat a variety of medical conditions and disorders. Implantable neural stimulation devices may be used in a spinal cord stimulation system. Implantable neural stimulation devices may also be used in deep brain stimulation, sacral nerve stimulators, cochlear implants or pacemakers.

Stimulation systems generally include an implantable neural stimulation device which typically includes a housing or body that encloses electronics/circuitry for generating the electrical pulses. The implantable neural stimulation device may be positioned under the skin by a medical professional. Stimulation leads are used to conduct the electrical pulses from the implantable neural stimulation device to a particular site to target the desired tissue. Electrodes on end of the stimulation leads are often used to deliver the electrical pulses to the desired tissue.

Implantable neural stimulation devices also comprise a header attached to the housing or body. The header may serve the purpose of receiving the stimulation leads as well as provide a point of communication or connection, such as connection for the electronics lead to the electronics/circuitry of the device.

Implantable neural stimulation devices of the prior art traditionally comprise one header. In this way the components of a charge coil, connectors and communication antenna(e) are included in the single header. Since they are all in one header this means that there is a limitation to the size of various components within such as the charge coil.

US8929986 discloses a stimulation device with one header, comprising both a charging coil and antenna.

US5313953 and US2010/019985 respectively disclose a stimulator with two headers, whereby one of the two headers contains a communication antenna and the other header contains at least one stimulation lead.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

Any discussion of documents, acts, materials, devices, articles or the like which has been included in the present specification is not to be taken as an admission that any or all of these matters form part of the prior art base or were common general knowledge in the field relevant to the present disclosure as it existed before the priority date of each claim of this application.

### Summary

The invention is defined by the device of independent claim 1 and by the method of independent claim 12. An implantable neural stimulation device comprising: a body containing stimulation electronics and a battery to provide stimulation energy; a first header coupled to the body of the device and containing a charge coil to charge the battery and a connector to connect a stimulation lead to the stimulation electronics to deliver the stimulation energy from the battery under control by the stimulation electronics; a second header coupled to the body and containing a communication antenna to provide communication between the stimulation electronics and an external communication device.

The body may include a pipe with open ends and the first header and second header may be attached to the body to close respective open ends. In some examples, this creates a hermetic container for the stimulation electronics and battery.

The device may comprise a first set of electronics leads to provide a link between the stimulation electronics and the first header.

The device may further comprise a second set of electronics leads to provide a link between the stimulation electronics and the second header. In some examples, the second set of electronics leads are located between the stimulation electronics and communication electronics.

The first set of electronics leads and/or the second set of electronics leads may be flexible leads. Flexible leads can be flexible flat cables and/or flexible printed circuits. The first set of electronics leads and/or the second set of electronics leads may be configured to collapse when the first header and the second header respectively are moved closer to the body.

The first set of electronics leads and the second set of electronics leads may be configured to collapse in a S-shape or Z-shape.

In some examples, at least one of the first set of electronics leads and/or second set of electronics leads are rigid leads.

In other examples, the link associated with the first set of electronics leads and/or second set of electronics leads further comprise one or more electrical connectors.

The first set of electronics leads and the second set of electronics leads may be coupled to the first header and second header respectively via a first feedthrough and a second feedthrough associated with the first header and the second header respectively, wherein the communications electronics are located between the second set of electronics leads and the second feedthrough.

The first header and the second header may be located at opposite sides of the body.

The communication electronics may be located proximal to the second header.

The communication antenna may be a radio frequency communication antenna. The communication antenna may be a two turn antenna.

A method of manufacturing an implantable neural stimulation device, the device comprising a body containing stimulation electronics, the body including a pipe with open ends, the device further comprising a first header and a second header, the first header comprising a first lid and the second header comprising a second lid, the method comprising: coupling a first set of electronics leads to provide a link between the stimulation electronics and the first header; coupling a second set of electronics leads to provide a link between the stimulation electronics and the second header; wherein the second set of electronics leads are located between the stimulation electronics and communication electronics; wherein at least the second set of electronics leads are flexible leads, and are configured to collapse when the first header and the second header respectively are moved closer to the body; and coupling the first header and the second header to the body to close respective open ends by; moving the first header and second header towards the body such that the first lid and the second lid close respective open ends; and forming a seal between the first lid, second lid and closed respective ends.

In the method, the communication electronics may be located proximal to the second header.

An implantable neural stimulation device comprising: a body containing: stimulation electronics; a communications electronics in communication with the stimulation electronics; one or more support elements; and a battery to provide stimulation energy; a first header coupled to the body of the device and containing a charge coil to charge the battery; and at least one connector to connect a stimulation lead to the stimulation electronics to deliver the stimulation energy from the battery under control by the stimulation electronics; a second header coupled to the body and containing a communication antenna to provide communication between the stimulation electronics and an external communication device; a second set of electronics leads to provide a link between the communications electronics and the communication antenna in the second header; wherein the one or more support elements are configured to isolate the second set of electronics leads from a surface of the body.

The one or more support elements may be configured to guide the second set of electronics leads to collapse in a consistent specified configuration when the second header is coupled to the body.

The at least one connector may be provided at the first header. The at least one connector may be provided at the second header.

The device may further comprise a first set of electronics leads to provide a link between the stimulation electronics and the first header.

An implantable neural stimulation device comprising: a body containing stimulation electronics and a battery to provide stimulation energy; a first header coupled to the body of the device and containing a charge coil to charge the battery; a second header coupled to the body and containing a communication antenna to provide communication between the stimulation electronics and an external communication device.

The body may further comprise a printed circuit board; a temperature sensor connected to the printed circuit board; and a rectifier connected to the printed circuit board.

An implantable neural stimulation device comprising: a body containing stimulation electronics and a battery to provide stimulation energy, wherein the body comprises a first portion and a second portion; a first header coupled to the first portion of the body and containing a charge coil to charge the battery; a second header coupled to the first portion of the body and containing a communication antenna to provide communication between the stimulation electronics and an external communication device; and a connector to connect a stimulation lead to the stimulation electronics to deliver stimulation energy from the battery under control by the stimulation electronics.

In some examples of the device, the stimulation electronics are provided at a printed circuit board contained in the body, wherein the printed circuit board has at least one notch for at least one set of electronics leads, that links the printed circuit board to a first or second header, to collapse in the notch.

In some examples of the device, the second header contains a further charge coil to charge the battery.

The first portion of the body and the second portion of the body may be welded together to form the body.

A method of manufacturing an implantable neural stimulation device, the device comprising a body with at least a first portion and a second portion to contain stimulation electronics and battery, a first header containing a charge coil, a second header containing a communications antenna, and at least one connector in the first and/or second header to connect a stimulation lead to stimulation electronics to deliver stimulation energy from the battery under the control of stimulation electronics. The method comprises: forming a first portion with at least one lid, wherein the lid has feedthroughs to couple with components of respective first and second headers; coupling the feedthroughs with at least the stimulation electronics and the battery; attaching the first portion of the body to the second portion of the body to seal the stimulation electronics and battery inside the body; and coupling respective first and second headers to the body, wherein components of the headers are in communication with components in the body via the feedthroughs.

In the method, coupling the first portion of the body and the second portion of the body to close respective open surfaces may comprise welding the first portion of the body and the second portion of the body.

In some examples, the method further comprises: locating a jig at, or relative to, the first portion to guide one or more flexible leads; and guiding one or more flexible leads between the feedthroughs and at least the stimulation electronics and battery, wherein the one or more flexible leads are guided by the jig to collapse in a consistent, specified configuration. Before the step of attaching the first portion of the body to the second portion of the body, the method further comprises removing the jig from the first portion.

A method of manufacturing an implantable neural stimulation device, the device comprising a body containing stimulation electronics, the body including a pipe with open ends, the device further comprising a first header and a second header, the first header comprising a first lid and the second header comprising a second lid, the method comprising: coupling a first set of electronics leads to form a link between the stimulation electronics and feedthroughs at the first lid; coupling a second set of electronics leads to form a link between the stimulation electronics and feedthroughs at the second lid, wherein the second set of electronics leads are flexible leads; preforming the second set of electronics leads with one or more mandrels to provide a preformed second set of electronics leads with one or more curves, wherein the preformed second set of electronics leads are configured to collapse in a consistent, specified configuration; and coupling the first header and the second header to the body to close respective open ends by: moving the first header and second header towards the body such that the first lid and the second lid close respective open ends, and collapsing the preformed second set of electronics leads in the consistent and specified configuration; and forming a seal between the first lid, second lid and the respective ends.

In some examples, the one or more mandrels comprise at least two mandrels, wherein the at least two mandrels preform at least part of the second set of electronics leads to an S-shape.

### Brief Description of Drawings

Fig. 1 illustrates an implantable neural stimulation device;
Fig. 2 illustrates a body of an implantable neural stimulation device;
Fig. 3 illustrates an example of a second header;

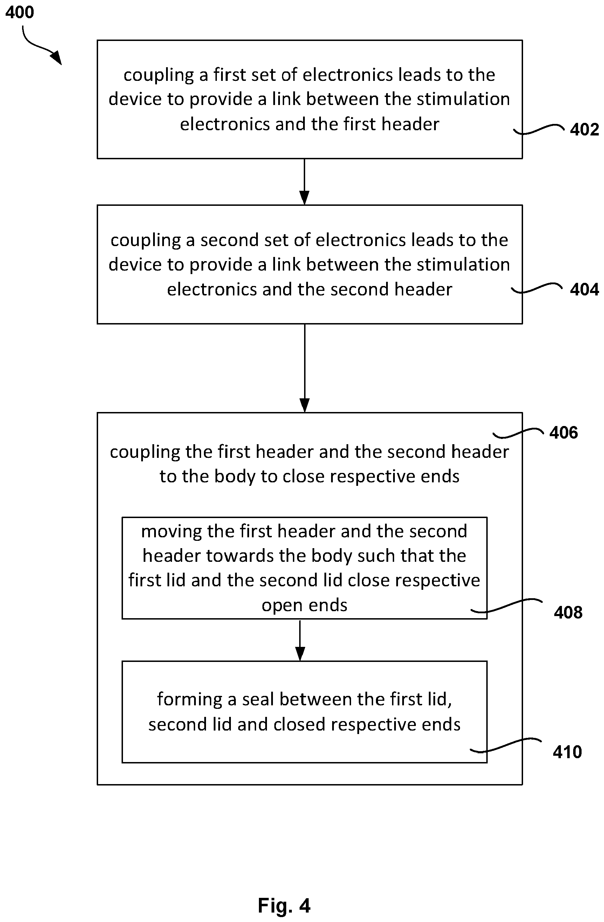

Fig. 4 illustrates a method of manufacturing an implantable neural stimulation device;
Figs. 5(a) to 5(b) illustrates an example of a configuration of the first and second set of electronics leads;
Fig. 6 illustrates a further example of a body for an implantable neural stimulation device with support elements for the electronic leads;
Fig. 7 illustrates a further example of an implantable neural stimulation device with connectors at a second header;
Fig. 8 illustrates a further example of an implantable neural stimulation device with connectors at both headers;
Fig. 9 illustrates a further example of a body for an implantable neural stimulation device;
Fig. 10 illustrates a further method of manufacturing an implantable neural stimulation device;
Fig. 11 is a schematic example of a body with a coupling having rigid feedthrough pins coupled to the PCB of the stimulation electronics;
Fig. 12 is a schematic example of a body with a coupling having a flex link connecting the feedthrough pins to the PCB of the stimulation electronics;
Fig. 13 is a schematic example of a body with a coupling having an electrical connector to connect the feedthrough pins to the PCB of the stimulation electronics;
Fig. 14 illustrates a schematic diagram of the internal components of another example of an implantable neural stimulation device;
Figs. 15(a) to 15(d) illustrate a sequence using a jig to align electronic leads during assembly of the implantable neural stimulation device;
Figs. 16(a) to 16(d) illustrate a sequence to preform electronics leads with a mandrel to provide consistent collapse of the electronics leads.
Fig. 17 is a top schematic view of another example with a support structure to support the PCB and support elements to guide electronics leads;
Fig. 18 is another view of the support structure and PCB of Fig. 17, with the electronics lead before assembly; and
Fig. 19 is another view of the support structure and PCB of Fig. 17 with the electronics lead assembled and guided by the support elements.

### Description of Embodiments

### Overview

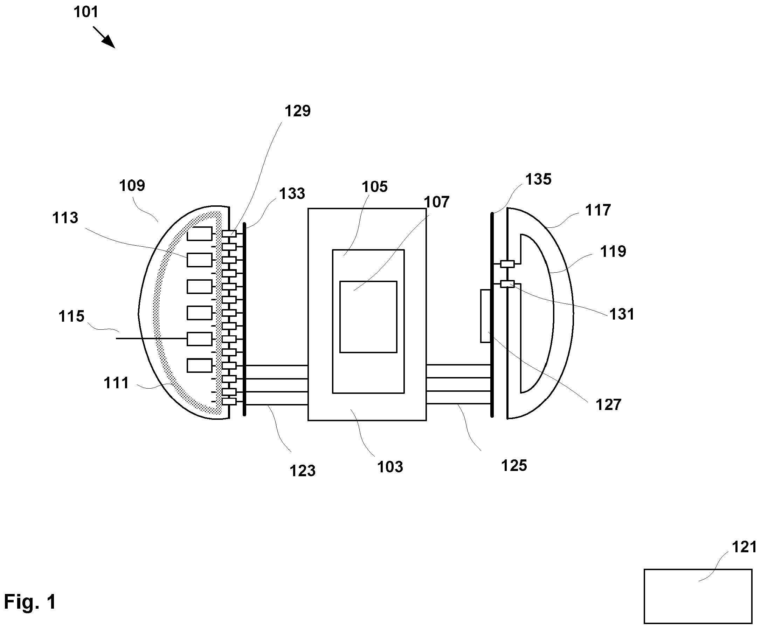

An example of an implantable neural stimulation device 101 is illustrated in Fig. 1. The device may be used in a spinal cord stimulation system. The device 101 comprises a body 103 containing stimulation electronics 105 and a battery 107 to provide stimulation energy. In some examples the stimulation electronics may be mounted on a printed circuit board.

The device 101 further comprises a first header 109 coupled to the body 103 of the device 101 and containing a charge coil 111 to charge the battery 107. The first header 109 further contains a connector 113 to connect a stimulation lead 115 to the stimulation electronics 105 to deliver the stimulation energy from the battery 107 under control by the stimulation electronics 105. The stimulation lead 115 may deliver the stimulation energy to a specific site to target desired tissue.

The device 101 further comprises a second header 117 coupled to the body 103 and containing a communication antenna 119 to provide communication between the stimulation electronics 105 and an external communication device 121. In some examples the communication antenna 119 is a radio frequency (RF) coil. The external communication device 121 may be associated with a medical professional.

Details of an exemplary implantable neural stimulation device 101 will now be described in detail

### Body 103 containing stimulation electronics 105 and a battery 107

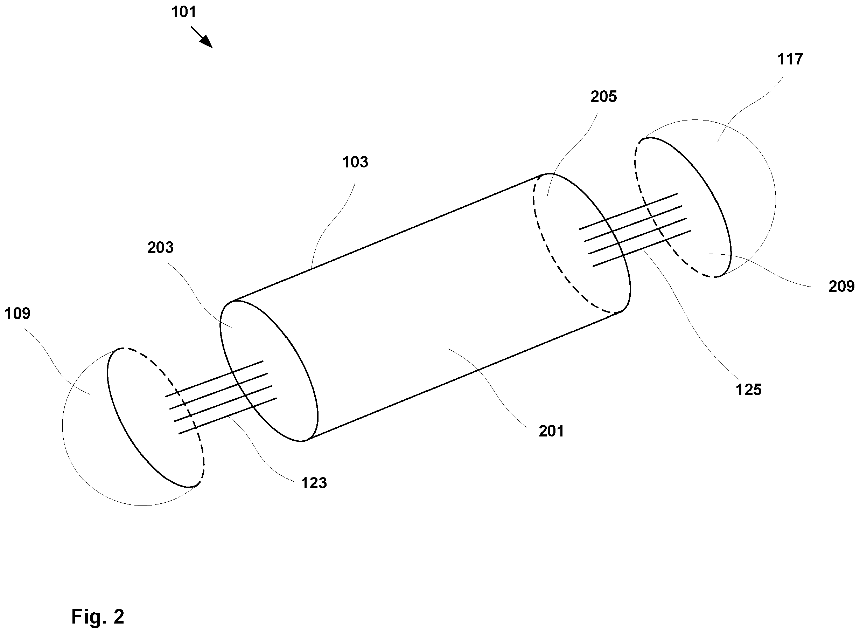

As described above the implantable neural stimulation device 101 comprisds a body 103 containing stimulation electronics 105. One example of a body 103 is illustrated in Fig. 2. In this example, the body 103 includes a pipe 201 with open ends 203, 205. The first header 109 and the second header 117 may be coupled to close respective open ends 203, 205.

The pipe 201 may be comprised of titanium. In another example the pipe may be comprised of a biocompatible ceramic. It is to be appreciated that other biocompatible materials may be used, including biograde stainless steels, biograde glass, silicon, alumina, zirconia, quartz, or metal alloys.

A first set of electronics leads 123 provides a link between the stimulation electronics 105 and the first header 109. A second set of electronics leads 125 provides a link between the stimulation electronics 105 and the second header 117. The body 103 and the open ends 203, 205 are configured to allow the first set of electronics leads 123 and second set of electronics leads 125 to pass through respective open ends 203, 205 during assembly.

The stimulation electronics 105 may comprise a microcontroller (uC) to: manage communications with programming systems and a patient remote control, manage charging of the rechargeable battery and other such housekeeping functions. The stimulation electronics 105 may further comprise a second microcontroller to manage patient therapy controlling an analogue only chip (AOC) to provide current sources and evoked response amplifiers. The stimulation electronics 105 may further comprise support components such as resistors, capacitors, power supplies and the like. The first microcontroller, second microcontroller and AOC may be packaged in ball grid arrays (BGA packages) to mount the components of the stimulation electronics 105 on to a printed circuit board 151. It is desirable to reduce the size of the stimulation electronics so that the size of the implantable pulse generator is minimised.

In some examples, the stimulation electronics 105 may be in communication with a communications electronics 153, such as a radio communications module. In some examples this may comprise a radio chip such as a radiofrequency (RF) chip. The communications electronics 153 may be connected to the printed circuit board 153 associated with the stimulation electronics 105. In this way, the second set of electronics leads 125 transmits analog communication signals to the communication antenna 119. In other examples the second set of electronics leads 125 may transmit digital communication signals.

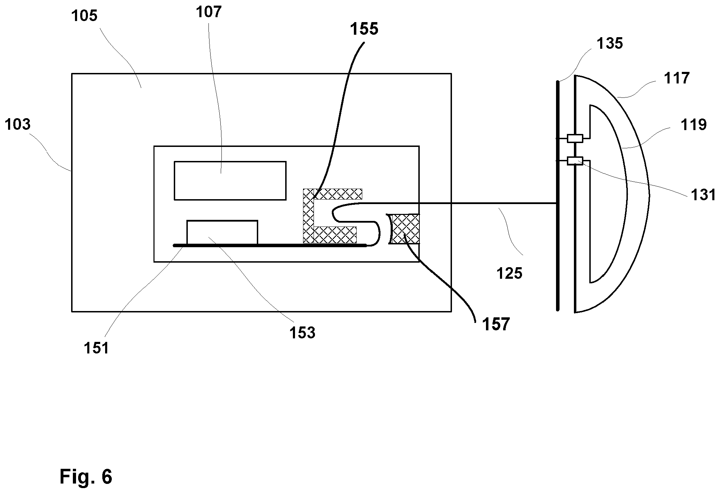

In some examples, the body 103 may comprise support elements 155, 157 as illustrated in Fig. 6. The support elements 155, 157 may be configured to isolate the second set of electronics leads 125 from a surface of the body 103. For example, the support elements 155, 157 may isolate the second set of electronics leads 125 from an inner surface of the body 103, such as an inner wall of the pipe 201. In a further example the support elements may isolate the second set of electronics leads 125 from the second lid 209. As illustrated in Fig. 6, the support elements 155, 157 prevent the second set of electronics leads 125 from contact with the lid 209 and the second set of electronics leads 125 may be in contact with the second header 117 via the second feedthrough 131. The support elements also function to ensure the second set of electronics leads fold (or otherwise collapse) in a consistent and specified configuration. This can be important for antenna matching with analog signals from the communications electronics.

It is an advantage that the communications electronics 153 may be connected to the printed circuit board 153 associated with the stimulation electronics 103. This reduces interference between the communications antenna 119 and the communications electronics 153.

A second advantage of having the communications electronics 153 connected to the printed circuit board 153 associated with the stimulation electronics 103 is provided by the ease of signal routing between the two modules. The signals between these are low-speed data signals and clock signals operating at approximately 1MHz and direct-current power signals. The routing for these signals is routine, as distinct to RF analog signals.

As described above the device 101 also comprises a battery 107 to provide stimulation energy. In one example the battery 107 is a rechargeable battery such as a lithium-ion battery or a lithium-ion polymer battery.

The charge coil 111 may be configured to charge the battery 107. In this way there may be an external charger or external charge coil that receives power and generates an electromagnetic field. The electromagnetic field generated by the external charge coil may induce an electrical current within the charge coil 111 to charge the battery 107 and thus provide stimulation energy to the stimulation electronics 105. The battery 107 may be recharged while the device is in use.

To provide the best energy coupling with the external charger or external charge coil, the charge coil 111 may be as large as possible. In this way the charge coil 111 may occupy a substantive space in the first header 109. It is an advantage of the present disclosure that the communications antenna 119 is located separate to the charge coil 111 in the second header 117 as this allows the charge coil 111 to occupy substantive space in the first header 109.

In one example, the charge coil 111 may comprise Litz strands.

The first header 109 and the second header 117 may be located at opposite sides of the body. An advantage of this is that interference between the charge coil 111 and the communications antenna 119 may be reduced.

### First header 109

As described above the device 101 further comprises a first header 109 coupled to the body 103. As illustrated in Fig. 1 the first header may have a rounded shape.

The first header 109 may comprise a charge coil 111 to charge the battery 107. The charge coil 111 may have a predetermined construction including a predetermined number of turns and diameter. For example the charge coil 111 may have 150 to 300 turns. In other examples the charge coil 111 may have 50 to 100 turns. The charge coil 111 may be comprised of magnetic wire. In one example the charge coil 111 may have an area of 386 sq mm, and have 58 turns each containing two strands of gold plated copper wire of 120um wire diameter.

The first header 109 may also comprise at least one connector 113 to connect a stimulation lead 115 to the stimulation electronics 105. Each connector 113 may have at least one associated simulation lead 115. In one example the connector 113 is a Bal Seal connector. Stimulation energy from the battery 107 may then be delivered via the connector 113 and stimulation lead 115. In one example the stimulation lead 115 may have one or more electrodes for contact with the desired tissue for treatment.

The device 101 may further comprise a first set of electronics leads 123 to provide a link between the stimulation electronics 105 and the first header 109. The first set of electronics leads 123 may comprise a balseal and charging flex link.In one example the balseal and charging flex link may be constructed of flexible PCB material having 26 wires, 24 being used to supply 2 x 12-channel electrode leads and the two remaining wires being used to connect to the charge coil 111.

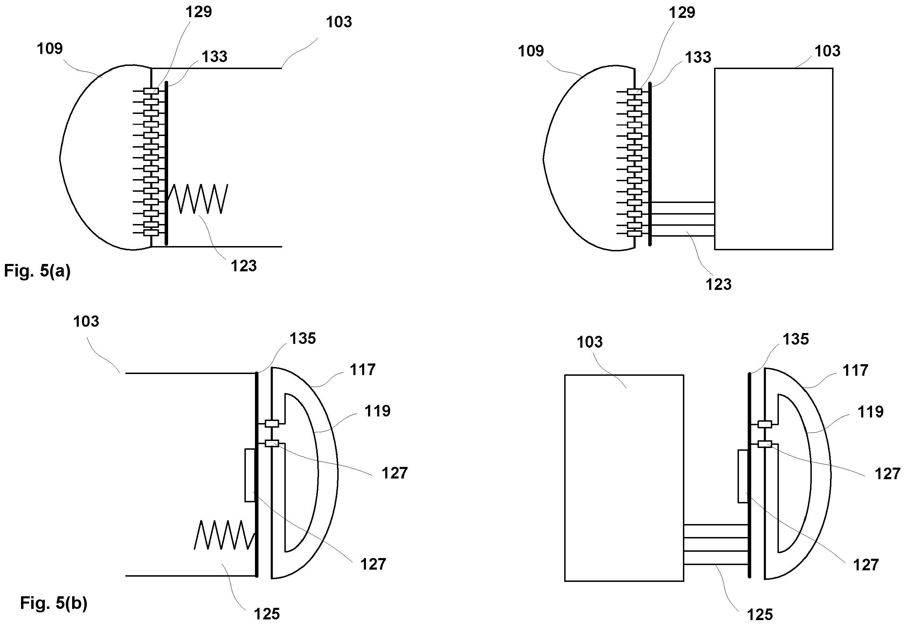

The first set of electronics leads 123 may be flexible leads. In this way, the first set of electronics leads 123 are configured to collapse when the first header 109 is moved closer to the body 103 of the device 101. The first set of electronics leads 123 may be configured to collapse in a S-shape or Z-shape. In this way, the first set of electronics leads 123 may be configured to concertina to a collapsed configuration. This is illustrated in Fig. 5(a). Similarly, the first set of electronics leads 123 are configured to extend when the first header 109 is moved away from the body 103. This is illustrated in Fig. 5(b).

The first set of electronics leads 123 may be coupled to the first header 109 via a first feedthrough 129 associated with the first header 109. The first set of electronics leads 123 may be coupled to the first header 109 via a plurality of feedthroughs. The first set of electronics leads 123 may further be connected to a first printed circuit board 133 of the device 101. This is illustrated in Fig. 1. The first feedthrough 129 may also be connected to the first printed circuit board 133. In this way the first set of electronics leads 123 may be coupled to the device 101 via the first printed circuit board 133 and the at least one first feedthrough 129.

The first header 109 may comprise a first lid 207. In this way, when the first header 109 is moved closer to the body 103 of the device 101 the first lid 207 closes and seals the first open end 203.

### Second header 117

As described above the device 101 further comprises a second header 117 coupled to the body 103. As illustrated in Fig. 1 the second header 117 may have a round shape.

The device 101 may further comprise a second set of electronics leads 125 to provide a link between the stimulation electronics 105 and the second header 117. As illustrated in Fig. 1, the second set of electronics leads 125 may be located between the stimulation electronics 105 of the body 103 and communication electronics 127.

In one example the second set of electronics leads 125 comprises a communications flex link. The communications flex link may be constructed of flexible PCB material having six wires, comprising power and ground (two wires) and a serial data bus (such as IIC) requiring four wires.

As illustrated in Fig. 1, the communications electronics 127 may be located proximal to the second header 117. In this way, the communications electronics 127 may be connected to a second printed circuit board 135 associated with the second header 117.

In one example, the communications module 127 may comprise an MICS band radio chip such as a ZL70103 mounted to a printed circuit board associated with the second header 117. The radio chip may be instead of, or in addition to, the communications electronics 153 associated with the printed circuit board 151 of the stimulation electronics 105 as described above. The printed circuit board may hold the antenna matching components and the power supply bypass capacitors. Since this has few components, it is able to fit into this small space.

The communications electronics 127 may comprise electronics that are suitable for wireless telemetry, such as radio frequency electronics. The communications electronics 127 may comprise one or more devices that provide a communications link between the device 101 and an external device 121, such as a chip or microcontroller. The communications electronics 127 may be used by an external device 121 or external service provider (such as a medical professional) to control various aspects of the device 101. The communications electronics 127 may also be used to receive data from, or send data to, the external device 121 or external service provider.

It is an advantage that the communications electronics 127 are located proximal to the second header 117. In this way the communications electronics 127 are located in a fixed distance to the communications antenna 119. The close proximity of the communications electronics 127 to the communications antenna 119 has the advantage of reducing signal/line loss and thereby improving communication between the device 101 and the external device 121 or external service provider.

In some variations, the second header 117 may not comprise communications electronics 127. In this way, communications electronics associated with the body 103 of the device 101, such as the communications electronics 153, may provide a communications link between the device 101 and an external device 121. That is, the communications electronics 153 may be used by an external device 121 or external service provider (such as a medical professional) to control various aspects of the device 101. The communications electronics 153 may also be used to receive data from, or send data to, the external device 121 or external service provider.

In some examples the interface to the communications electronics 127 may be one or more leads from the second set of electronics leads 125.

The second set of electronics leads 125 may be flexible leads. In this way, the second set of electronics leads 125 are configured to collapse when the second header 117 is moved closer to the body 103 of the device 101. The second set of electronics leads 125 may be configured to collapse in a S-shape or Z-shape. In this way, the second set of electronics leads 125 may be configured to concertina to a collapsed configuration. This is illustrated in Fig. 5(a). Similarly, the second set of electronics leads 125 are configured to extend when the second header 117 is moved away from the body 103. This is illustrated in Fig. 5(b).

The second set of electronics leads 125 may be coupled to the second header 117 via a second feedthrough 131 associated with the second header 117, such that the communications electronics 127 is located between the second set of electronics leads 125 and the second feedthrough 131. The second set of electronics leads 125 may be coupled to the second header 117 via a plurality of feedthroughs.

The second set of electronics leads 125 may be connected to the second printed circuit board 135 of the device 101. This is illustrated in Fig. 1. The second feedthrough 131 may also be connected to the second printed circuit board 135. In this way the second set of electronics leads 125 may be coupled to the device 101 via the second printed circuit board 135 and the at least one second feedthrough 131.

The second set of electronics leads 125 may provide a pathway for the serial data and power communications of the device 101.

As indicated above the second header 117 comprises a communication antenna 119. The communication antenna 119 may be a radio frequency (RF) communication antenna. The communication antenna 119 may be a two-turn antenna. The communication antenna 119 may be a multi-turn antenna. An advantage of this is that the second header 117 may be reduced in height compared to the first header 109.

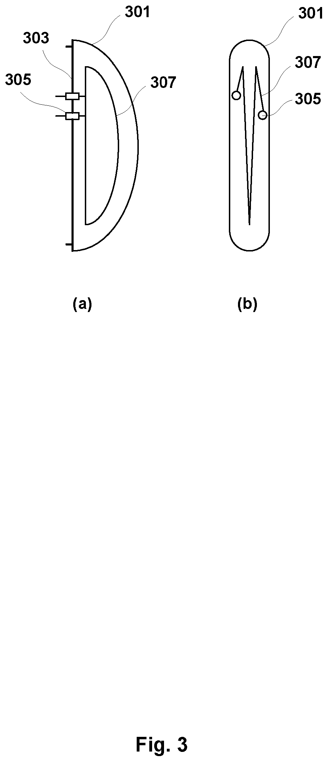

An example of the second header 117, 301 is illustrated in Fig. 3.

The second header 117 may comprise a second lid 209. In this way, when the second header 117 is moved closer to the body 103 of the device 101 the second lid 209 closes and seals the second open end 205.

### Method 400 of manufacturing an implantable neural stimulation device

There is also provided a method 400 of manufacturing an implantable neural stimulation device 101, the device 101 comprising a body 103 containing simulation electronics 105, the body 103 including a pipe 201 with open ends 203, 205. The device 101 may further comprise a first header 109 and a second header 117, the first header 109 comprising a first lid 207 and the second header 117 comprising a second lid 209.

The method 400 may further comprise coupling 402 a first set of electronics leads 123 to the device 101 to provide a link between the stimulation electronics 105 and the first header 109. Coupling 402 may comprise establishing an electrical connection between the first set of electronics leads 123 and the stimulation electronics 105.

The method 400 may further comprise coupling 404 a second set of electronics leads 125 to the device 101 to provide a link between the stimulation electronics 105 and the second header 117. Coupling 404 may comprise establishing an electrical connection between the second set of electronics leads 125 and the stimulation electronics 105. The second set of electronics leads 125 may be located between the stimulation electronics 105 and communications electronics 127. In this way, electrical connection may be established between the stimulation electronics 105, the second set of electronics leads 125 and the communications electronics 127. A communications connection (such as radio frequency) may further be established between the second set of electronics leads 125 and the communications electronics 127.

The first set of electronics leads 123 and second set of electronics leads 125 may be flexible leads, and may be configured to collapse when the first header 109 and the second header 117 respectively are moved closer to the body 103. Therefore during assembly, the first and/or second electronics leads 123, 125 may be stretched out via the open ends 203, 205 to aid ease of connecting the feedthroughs 129, 131 to respective first and second printed circuit boards 133, 135. When the headers 109, 117 are moved to close the open ends 203, 205 the electronics leads 123, 125 concertina inside the body 103.

The method 400 further comprises coupling 406 the first header 109 and the second header 117 to the body 103 to close and seal respective open ends 203, 205.

Coupling 406 comprises moving 408 the first header 109 and the second header 117 towards the body 103 such that the first lid 207 and the second lid 209 close respective open ends 203, 205. The method 400 further comprises forming 410 a seal between the first lid 207, second lid 209 and closed respective ends 203, 205.

In the method 400 of manufacturing the device 101, the communication electronics 127 may be located proximal to the second header 117. In this way, the communications electronics 127 may be connected to the second printed circuit board 135.

### Variations

As described above, the first connectors 129 of the first header 109 may comprise at least one Bal Seal connector. In some examples, the second header 117 may comprise connectors 113. In further examples the second header 117 may comprise at least one Bal Seal connector.

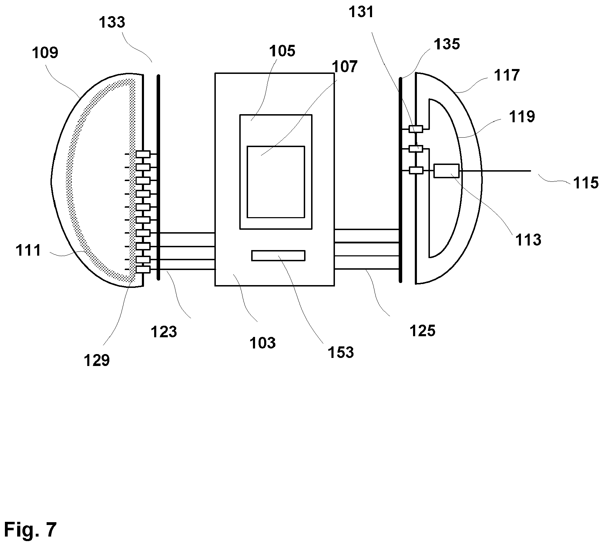

In a further example of an implantable neural stimulation device 101, the first header 109 may solely comprise the charge coil 111. This is illustrated in Fig. 7. In this example the second header 119 may comprise at least one connector 113 to connect a stimulation lead 115 to the stimulation electronics 105 to deliver the stimulation energy from the battery 107 under control by the stimulation electronics 105.

An advantage of this example is that the charge coil 111 can have a higher charging efficiency due to lower interference by metallic elements, such as connectors 113. The first header 109 may also assume a rounded shape as the shape of the charge coil 111 may be modified with minimal or no effect to the charging efficiency of battery 107.

A further advantage is that the number of turns in charge coil 111 may be increased. This may result in better charge coupling of battery 107 and thus better provision of stimulation energy to the stimulation electronics 105.

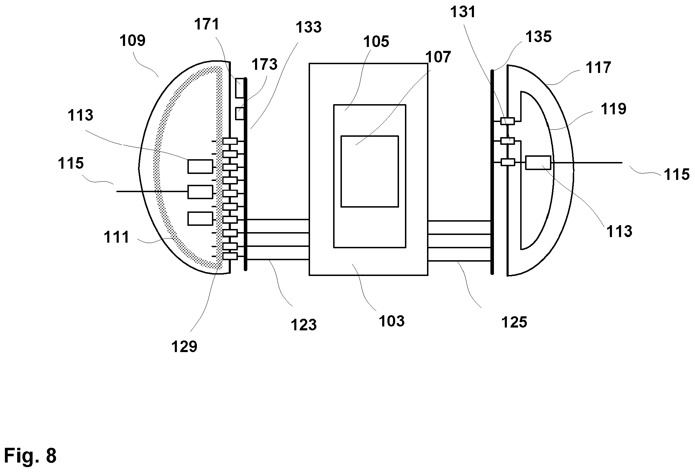

As illustrated in Fig. 8, the first header 109 may comprise a first printed circuit board 133. The first printed circuit board 133 may be positioned on the first lid 207. In this example, the first printed circuit board 133 may be connected to a temperature sensor 171. The temperature sensor 171 may detect anomalous operation of device 101.

In a further example, the first printed circuit board 133 may also be connected to a rectifier 173. The rectifier 173 may comprise a bridge rectifier to output a DC voltage to the stimulation electronics 105. An advantage of this example is that the rectifier 173 may reduce operating noise of the device while charging, as the highfrequency components (above 425 kHz) are eliminated at the rectifier.

A further advantage is that the number of strands in the charging coil 111 may be increased. In one example the number of Litz wire strands in the charge coil 111 may be increased from two to three or more. In this way, the increased Litz wire strands in the charge coil 111 reduce Skin Effect losses during charging. The increased Litz wire strands in the charge coil 111 may also lower the resistance of the charge coil 111.

### Further variation of the device 101

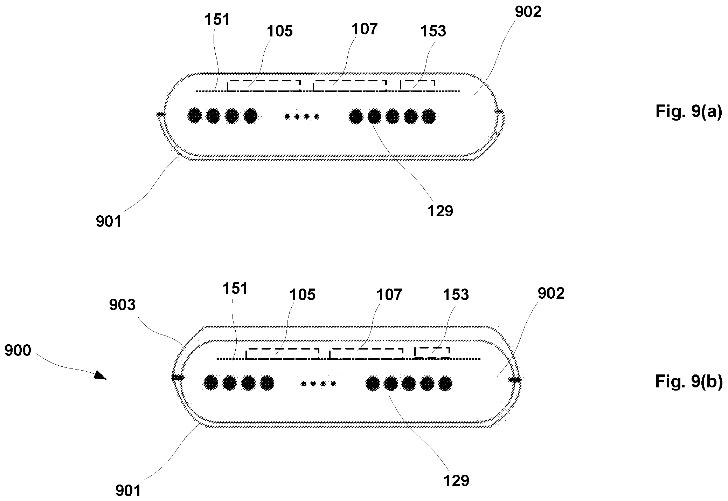

Figs. 9(a) and 9(b) illustrate a further example of an implantable neural stimulation device 101. This example device 101 comprises a body 900 containing stimulation electronics 105 and a battery 107 to provide stimulation energy. In some examples the stimulation electronics may be mounted on a printed circuit board 151. The body 103 may further comprise communications electronics 153. These are illustrated in Figs. 9(a) and (b) in broken lines to indicate they are behind the lid 902 as will be discussed below.

As illustrated in Fig. 9 body 900 of the device 101 comprises a first portion 901 and a second portion 903. The first portion 901 and second portion 903 (and lid 902) are attached together to form a sealed body 900 to contain the stimulation electronics and battery 107. This may include welding the first portion 901 and second portion 903 to each other.

The first portion 901 formed with one or more lids 902. In some examples, the lid 902 may be integrally formed with the first portion 901. In other examples, the lid 902 is initially a separate component welded (or otherwise attached) to the first portion 901. The lid 902 has feedthroughs 129 to couple with components of respective first and second headers (not shown in Figs. 9 but described in earlier examples above) with components inside the body 900.

The first header 109 comprises a charge coil 111 to charge battery 107. The second header 117 comprises a communication antenna 119 to provide communication between the stimulation electronics 105 and an external communication device 121. In some examples the second header 117 may further comprise communications electronics 127.

The first header 109 and/or the second header 117 further comprises a connector to connect a stimulation lead 115 to the stimulation electronics 105 to deliver the stimulation energy from the battery 108 under control of the stimulation electronics 105. The connector in the header(s) are, in turn, in communication with components inside the body 900 via the feedthroughs 129.

A first set of electronics leads 123 are coupled to the first header 109 via the feedthroughs 129. In this way the first set of electronics leads 123 provides a link between the stimulation electronics 105 and the first header 109. A second set of electronics leads 125 are coupled to the second header 117 via feedthroughs 129 (on an opposite side). In this way the second set of electronics leads 125 provides a link between the stimulation electronics 105 and the second header 117.

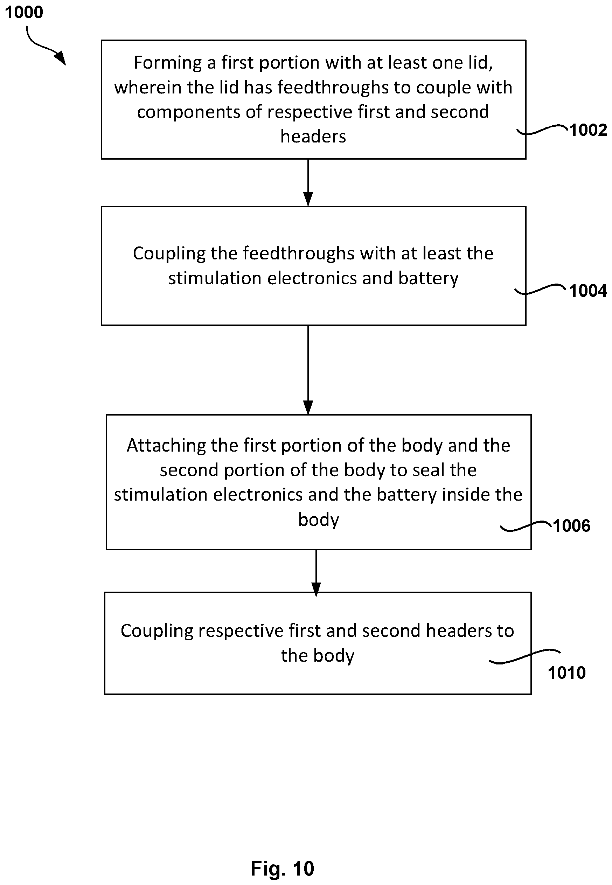

Fig. 10 illustrates a method 1000 of manufacturing an implantable neural stimulation device 101, the device 101 comprising a body 900, the body 900 including a first portion 901 and a second portion 903. The device 101 further comprises a first header 109 containing a charge coil and a second header 117 containing a communications antenna. At least one connector is provided in the first and/or second header to connect a stimulation lead to stimulation electronics to deliver stimulation energy from the battery under the control of stimulation electronics.

The method 1000 includes forming 1002 a first portion 901 with at least one lid 902 (as illustrated in Fig. 9(a)). This may include welding (or otherwise attaching) a lid 902 to a first portion 901 or alternatively integrally forming the first portion with a lid. The lid has feedthroughs 129 to couple with components of the respective first and second headers.

The method further comprises coupling 1004 the feedthroughs 129 with at least the stimulation electronics 105 and battery 107. Coupling 1004 may comprise establishing an electrical connection between the feedthroughs 129 and the stimulation electronics 105. Examples of couplings will be described in further detail below and can include variations, combinations and permutations of rigid feedthroughs, flexible leads/links (cables?); and/or electrical connectors.

The method 1000 further comprises attaching 1006 the first portion 901 of the body 900 to the second portion 903 of the body 900 to seal the stimulation electronics and battery inside the body 900 (as illustrated in Fig. 9(b)).

The method 1000 further includes coupling 1010 the first and second headers to the body so that components of the headers are in communication with respective components in the body via the feedthroughs 129. In some examples, the headers are coupled to the lid(s) 902 of the sealed body 900. This may include coupling the headers with epoxy or welding to provide a seal between the headers and body 900.

It is to be appreciated that in some alternative examples, the headers can be attached to the lid(s) and/or the first portion 901 before the step of attaching 1006 the first portion 901 to the second portion 903s.

### Examples of couplings

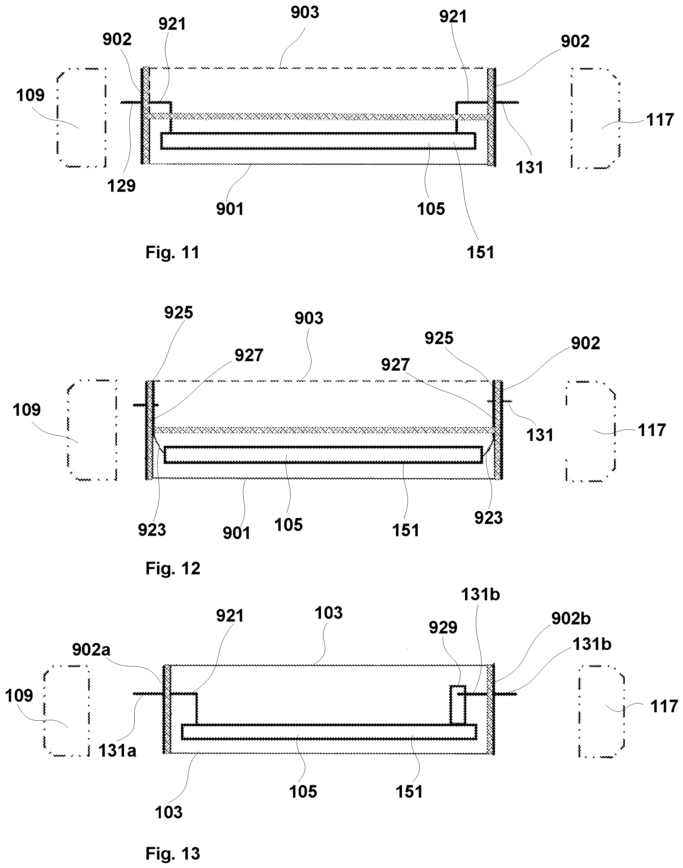

Examples of couplings to establish electrical connections between feedthroughs and the stimulation electronics 105 will now be described with reference to Figs. 11 to 13.

Fig. 11 illustrates a schematic where the feedthrough pins 131 have a rigid extension 921 to electrically connect with the PCB 151 of the stimulation electronics 105. In some examples, a continuous, rigid, and electrically conductive feedthrough pin 131 is soldered (or otherwise coupled) directly to the PCB 151.

In one example, the assembly initially includes having a lid 902 attached with the rigid feedthrough pins 902 and rigid extensions 921. The lid 902 is then positioned towards the first portion 901, that includes positioning the extensions 921 to the PCB 151. The rigid extensions 921 are then attached (such as with soldering) to the PCB 151 and the lid 902 welded to the first portion 901. This can be done for the lids 902 at either ends of the first portion 901. The second portion 903 (shown in dash lines) can then be welded to the first portion 901 and lids 902. Finally, the headers 109, 117 can be formed at the lids 902.

It is to be appreciated that the above steps can be varied. For example, the lid 902 can be welded to the first portion 901, and then subsequently, the rigid extensions 921 attached to the PCB 151. In yet another example, the first portion 901 and lid 902 are initially free of the PCB 151, feedthrough pins 131 and extensions 921. The first portion 902 and lids 902 are welded together, and subsequently the PCB 151, feedthrough pins 902 and rigid extensions 921 are located and fixed in place (before the second portion 903 is mated to the assembly).

It is to be appreciated that in other embodiments, the feedthrough pins 131 and rigid extensions 921 are initially separate components, and that during assembly the feedthrough pins 131 and rigid extensions 921 are soldered, or otherwise rigidly connected to one another.

Fig. 12 illustrates a schematic of another example where the feedthrough pins 131 are coupled to the PCB 151 via, at least in part, a flexible leads 923. In one example, the lid 902 has attached feedthrough pins 131, whereby the lid 902 is brought in proximity to the first portion 901. The flexible leads 923 are then soldered (or otherwise attached) to form an electrical connection between the PCB 151 and the feedthrough pins 131. The lid 902 is then positioned and welded to the first portion 901. Other components such as the second portion 903 and the headers 109, 117 can be attached as described in earlier examples.

In some examples, the lid has an intermediate component 925 between the feedthrough pins 131 and the flexible leads 923. The intermediate component 925 can include a further printed circuit board with one or more conductive tracks 927. The feedthrough pins 131 and flexible leads 923 can be soldered or otherwise attached to these tracks 927 to complete the electrical connection. The intermediate component 925 can be configured to abut with the lid 902, which can improve strength and rigidity to the components. Although not illustrated in Fig. 12 for clarity, it is to be appreciated that the flexible leads 923 can be configured to collapse with one or more of the configurations described in this specification, including the use of support elements, notch, and/or jig (described in further detail below).

Fig. 13 illustrates a schematic of yet another example where some of the feedthrough pins 131 are coupled, via a electrical connector 929, to the PCB 151. In some examples, the electrical connector 929 is mounted, and electrically coupled, to the PCB 151. The electrical connector 929 is configured to receive feedthrough pins 131 to which respective electrical connections are made. In some examples, the feedthrough pins 131 are push fit into receptacles (such as sockets) in the electrical connector 929.

In one example, the body 103 is substantially tubular with open ends that can be closed by respective lids 902a and 902b. At a first end, feedthroughs 131a are provided at the lid 131a and, via extension 921 (or alternatively flexible leads 923) electrically coupled to the PCB 151 in a similar way to examples described in Figs. 11 and 12. The first lid 902b is then positioned towards the body 103 such that the connected PCB 151 slides into the body 103. The second lid 902b has feedthroughs 131b and as the second lid 902b is brought towards the opposite end of the body 103, the feedthroughs 131b are received by the electrical connector 929 to complete the electrical coupling. The lids 902a and 902b can then be attached (such as by welding) to the body 103.

It is to be appreciated that these steps can be reordered. For example, the second lid 902b can be attached to the body 103 before the first lid 902a and PCB 151 are slid into the body 103. In another example, the first lid 902a can be attached to the body 103 before the second lid 902b is brought towards the body 103.

In yet further examples, the body 103 in Fig. 13 can be constructed with two or more portions (like the example in Figs. 9(a) and 9(b)). This can include positioning one or more lids 902a, 902b, and the PCB 151, before the first and second portions 901, 903 (or other portions) are assembled together.

In some examples, using rigid extensions 921 and/or electrical connectors 929 can be advantageous in providing a predictable and/or repeatable electrical path to the communication antenna 119. This can facilitate antenna matching compared to a flexible lead between communication electronics 127 and the communication antenna 119. Thus in some examples, the communication electronics can be located on PCB 151, whereby the electrical path to the communication antenna 119 is via rigid extensions 921 and/or electrical connectors 929 (and without flexible leads).

It is to be appreciated that various combinations and permutations of the above couplings can be used in other variations.

### Further variation of the device with notch in the printed circuit board

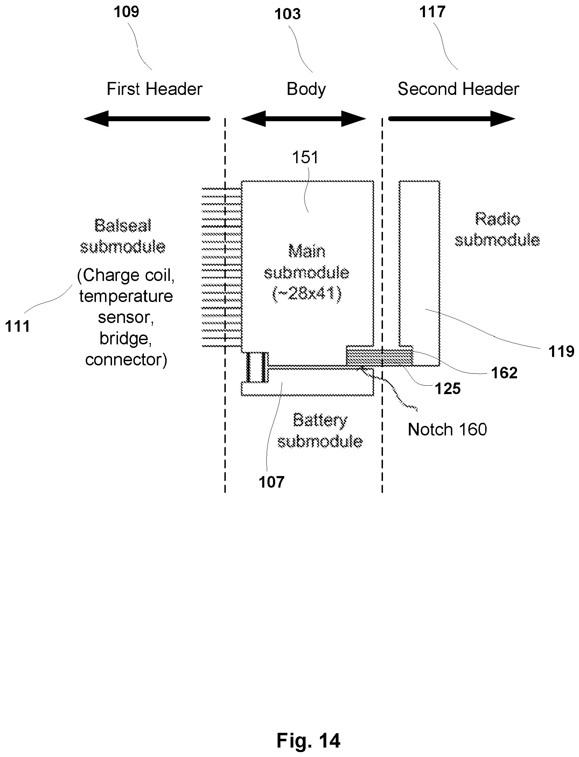

Fig. 14 illustrates internal components of yet another example of the implantable neural stimulation device. In this example, the printed circuit board 151 located in the body 103 has a notch 160 to provide space and clearance for the flexible leads 125 to fold when assembled. In some further examples, the notch 160 also provide space for support elements 155, 157. Having a notch 160 may maximise the space on the PCB for other electronic components.

In some examples, another notch 162 is provided at the header 117 (and/or 109) to provide additional space and clearance for the flexible leads 125. This may be in conjunction with the notch 160 or, in some examples, as an alternative.

In examples where the notch 160 is predominantly at the PCB 151 in the body 103, this can maximise the space in the header(s) 109, 117 for other components. For example, maximising the space in the header for more strands for a charge coil 111 or antenna 119.

An example of assembling the device will now be described. The electrical and electronic components of the first header 109 are soldered to respective components of the PCB 151 and/or battery 107. This may be done whilst the PCB 151 is separated from the body 103. Without the body, it is easier for a person or machine to solder the multiple flexible leads. The body 103, such as a metal sleeve, can then be slid over the PCB 151 and towards the first header 109. The body 103 may then be sealed against the first header 109, such as by welding or epoxying. Next, the flexible leads 125 are then soldered to link the PCB 151 with components in the second header 117 (such as an antenna 119 or communications electronics). The flexible lead 125 can be provided with some additional length to allow room for a person or machine to solder (since the body 103 will reduce ease of access to solder to the PCB/second header). The second header 117 is then brought towards the body 103 in a controlled manner to allow the flexible leads 125 to fold/collapse in a consistent manner (e.g. to concertina the leads). This is assisted by the notch 160 that provides room for the flexible leads 125 to fold/collapse. The second header 117 can then be sealed against the body 103 by welding or epoxying.

It is to be appreciated that in some examples the first header 109 may be sealed against the body 103 at later stages - such as around the same time the second header 117 is sealed.

In another variation, a further charge coil is located in the second header 117.

### Further variation of a method of manufacture a stimulation device using a jig

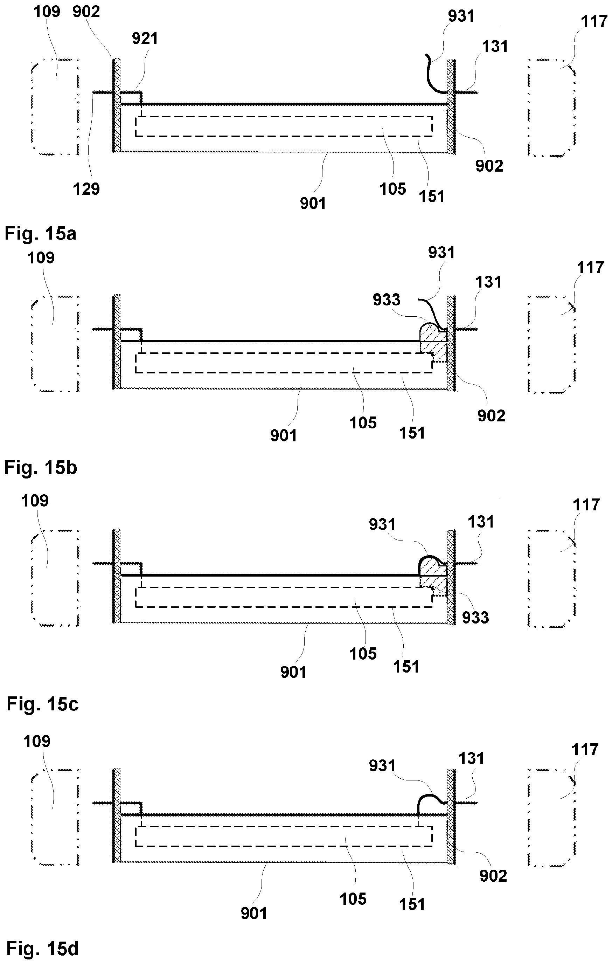

Figs. 15(a) to 15(d) illustrate a sequence to manufacture an implantable neural stimulation device 101. In this example, the body of the device 101 comprises at least a first portion 901 and a second portion 903 (not shown in Figs. 15 but illustrated in Fig. 9(b)). The method includes forming a first portion 901 with one or more lids 902, where the lid 902 has feedthroughs 129, 131.

In Fig. 15(a), the right hand feedthrough 131 is connected to flexible lead 931, whereby the flexible lead 931, in turn, is to be connected to the stimulation electronics 105/PCB 151 (and/or battery). In one particular example, the flexible lead 931 connects to communication electronics on the PCB 151 (or other component mounted to the body).

In Fig. 15(b), a jig 933 is located at the first portion 901 to guide the flexible lead 931. In some examples, this includes placing the jig 933 in abutment to at least part of the first portion 901. In other examples, this may include placing the jig 933 relative to the first portion 901 via an intermediate part. For example, the PCB 151 can be mounted to the first portion 901 and, in turn, the jig 933 is placed in abutment with the PCB 151.

In Fig. 15(c), the flexible lead 931 is guided to the stimulation electronics 105/PCB 151 (and/or battery) with the jig 933 so that the flexible lead 931 collapses in a consistent and specified configuration. The flexible lead 931 is then connected to the stimulation electronics/PCB/battery by soldering, or other attachment system. This consistent and specified configuration can ensure the flexible lead 931 is not collapsed in another configuration that could inadvertently bend and cause the flexible lead 931 to weaken. It can, in cases where the flexible lead 931 is used for the communication antenna, provide predictable electromagnetic characteristics for the antenna.

In Fig. 15(d), the jig 933 is removed from the first portion 901. This figure also illustrates the flexible lead 931 that maintains the specified configuration caused by the jig 933. Subsequently, a second portion 903 of the body can be attached to the first portion to seal the stimulation electronics and battery inside the body (using techniques as described in earlier examples).

In the above example, the left hand side feedthrough 129 is linked to the stimulation electronics 105/PCB 151 via a rigid extensions 921. It is to be appreciated that in other examples, combinations and variations with other coupling techniques can be used, including using a jig 931 to guide flexible leads to both lids.

### Further variation to preform electronics leads

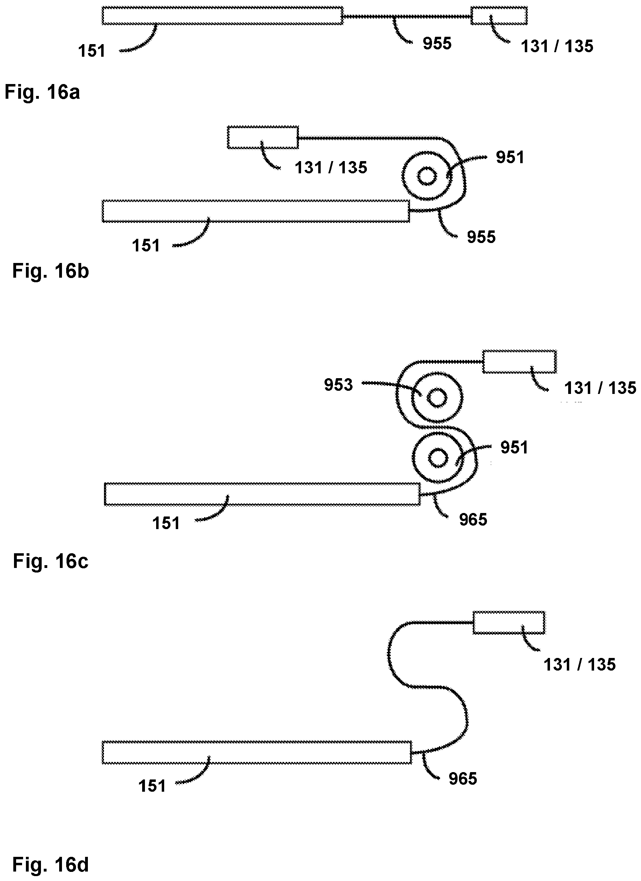

Figs. 16(a) to 16(d) illustrates a sequence to preform a set of electronics leads 955 with a mandrel 951, 953. By manipulating the set of electronics leads 955 to a preformed set of electronics leads 965, this can assist the electronics leads 965 to collapse in a consistent, specified and predictable configuration. This can be important for electronics leads where the shape and configuration can affect performance, such as those associated with an antenna.

This approach can be applied to one or more of the neural stimulation devices described herein, including the examples where the body of the device comprises a pipe. For brevity, we have only illustrated the pertinent aspects of this variation in Figs. 16 and, to avoid doubt, other features described herein can be used with this variation.

Fig. 16(a) illustrates a PCB 151, which can support stimulation electronics, that is coupled to feedthrough 131 (and/or second PCB 135 associated with a header) via a set of electronics leads 955. In this example, the electronics leads are flexible leads such that they can bend (at least to a threshold) without breaking.

Referring to Fig. 16(b) a mandrel 951, such as a cylinder, is provided and the set of electronics lead 955 wrapped around at least part of the mandrel 955 to form a first curve on the electronics lead 955.

In Fig. 16(c), an additional mandrel 951, is provided to preform a second curve, resulting in a preformed set of electronics lead 965 with an S-shape. It is to be appreciated that other shapes, in addition to S-shapes can be used in accordance with a desired design shape. For example, a "W-shape" or "U-shape" may be used.

In Fig. 16(d) the mandrels 951, 953 are withdrawn. In some examples, the electronics lead 965 may be formed against the mandrel 951, 953 past the yield point such that the electronics lead 965 is deformed plastically and maintains (or substantially maintains) the shape (such as the S-shape). In other examples, the electronics lead 965, after preforming, is elastically deformed and requires other elements to support and maintain the preform shape.

After producing the preformed set of electronics leads 965, the other components of the device can be assembled. In particular, moving the lids with the feedthroughs 131/second PCB 135 towards the pipe body. As the lids move closer towards the ends of the pipe, the preformed set of electronics leads collapse in a consistent and specified configuration dictated by the preformed shape. The lids can then be sealed against the ends of the pipe.

It is to be appreciated that different variations and combinations can be used. For example, the first header and lid may have a first set of rigid electronics leads that are soldered rigidly to the PCB 151 (as described as rigid extensions 921 in Fig. 15(a)). The PCB 151 is then inserted through the pipe with the lid of the first header closing one end of the pipe. A second set of flexible electronics leads 955 are then connected between the PCB 151 and feedthroughs 131 of the second header. The mandrels are then positioned and the second set of electronics leads 955 preformed into a desired shape. The mandrels are then withdrawn and the lid of the second header moved towards the remaining open end of the pipe. During this movement, the preformed second set of electronics lead 965 collapses in a consistent and specified manner inside the pipe. Finally, the lid of second header is sealed with the pipe.

In yet another example, both the first set of electronics leads and the second set of electronics leads are preformed by mandrels.

In some examples, the mandrels are substantially cylindrical in shape. The mandrels can include rollers. In other examples, the mandrel can be frustoconical. In yet further examples, the mandrel may be a prism, such as a rectangular prism, pentagonal prism, hexagonal prism, heptagonal prism, etc.

### Variation with support structure to support PCB

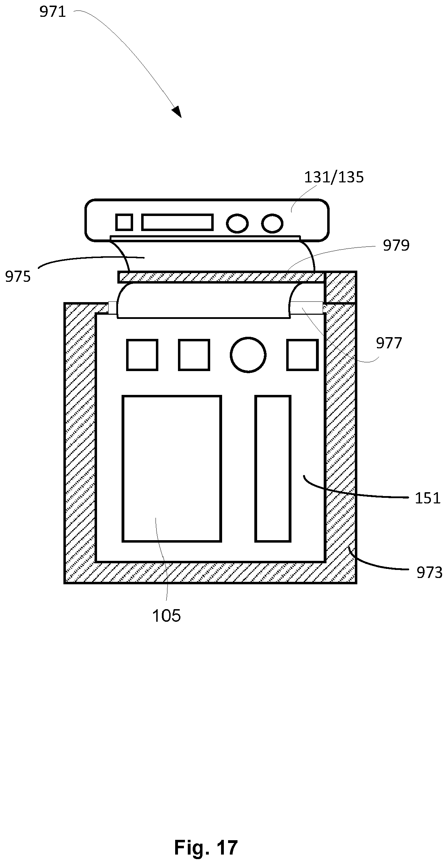

Figs. 17 to 19 illustrate another example 971 with a support structure 973 to support the PCB 151 inside the body 103. The support structure 973, in one example, includes an moulded structure that receives the PCB 151, which in turn is received snugly in the body 103 (not shown in Figs. 17 to 19, but illustrated in earlier embodiments). This can reduce the likelihood of damage to the PCB from movement and vibration against the body 103, when assembled. In some examples, the support structure 973 provides thermal and/or electrical insulation between the PCB 151 and the body 103. The support structure 973 made be made of polymer. The support structure 973 can be assembled with the PCB 151, after which the support structure 973 and PCB 151 can be slid into the body 103.

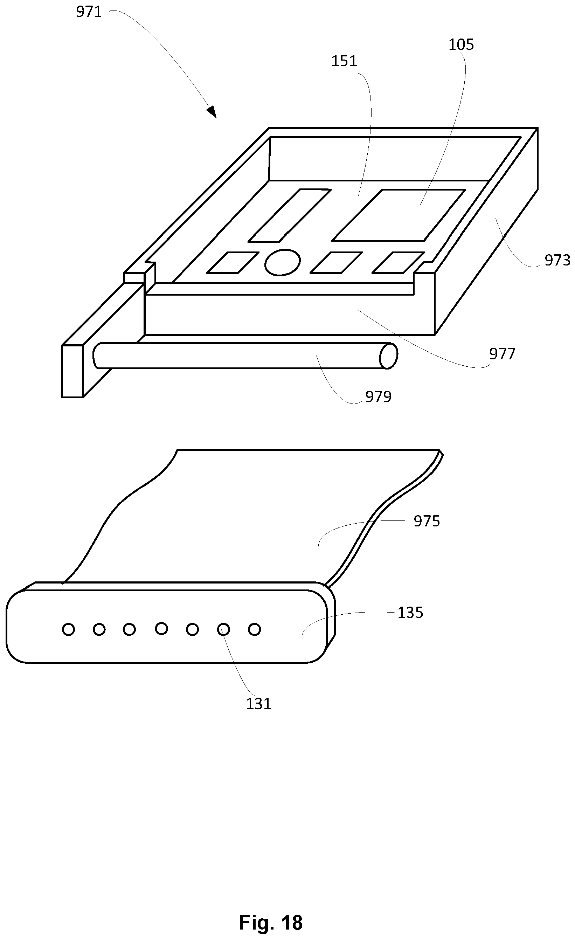

The support structure 973 further comprises one or more support elements 977, 979 to guide corresponding (flexible) electronics leads 975. Referring to Fig. 18, a first support element 977 is formed from a notch in a perimeter wall surrounding the PCB 151. A second support element 979, in this example, includes a protrusion in the form of a cylindrical finger.

The electronics lead 975, in the form of a flexible lead 975 is electrically connected to the feedthroughs 131. In some example, this can be through an intermediate second printed circuit board 135 associated with a lid of a header.

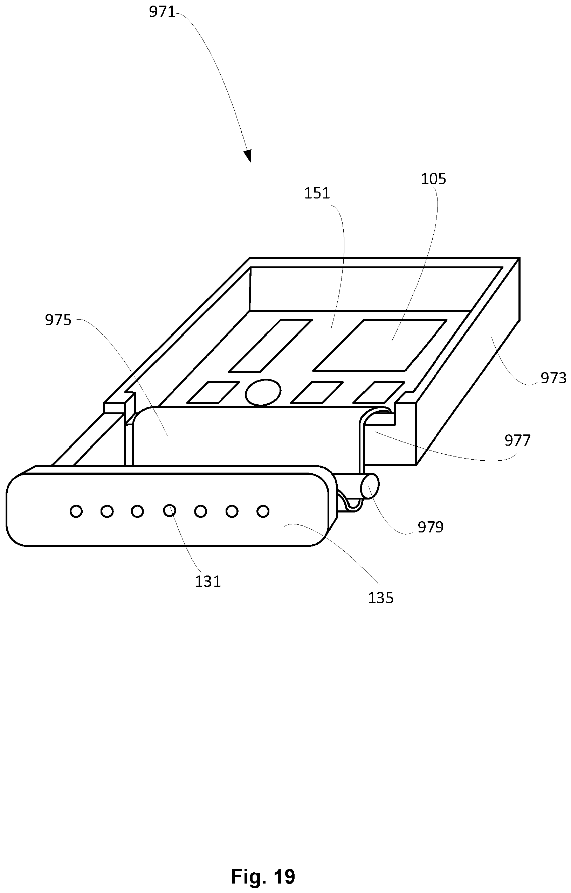

Assembly of the electronics lead 975 to the PCB 151 is illustrated in Fig. 19 where the electronics lead 975 is guided by the support elements 977, 979 in an S-shape. This allows the electronics lead 975 to fold/collapse in a consistent matter. This aids in assembly of the device which can include bringing opposing lids (of respective headers) together in a pipe body 103 as described above..

It is to be appreciated that in some examples, only one support element is used, whilst in others, three or more support elements are used. Furthermore, it is to be appreciated that the support elements can be configured to assist the electronics leads 975 to collapse in other desired shapes that are consistent and predictable.

In yet further examples, the one or more support elements are configured to bend, pivot, move and/or otherwise displace in specified manner. For example, the support element 979, in the form of the protrusion can be hinged to the remainder of the support structure 973. Such a hinge can provide a predictable path for the support element 979 to fold or collapse with the supported electronics lead. Other forms of a movable support element can include a support element moving along a track in the support structure. In further examples, the support element 979 is biased by a spring mechanism that can reduce blacklash (i.e. unwanted movement).

In this illustrated examples, only one set of flexible electronics leads 975 are illustrated for brevity. It is to be appreciated that another set of electronics leads 975 are provided on the opposite side for the other header, and may include flexible leads, rigid leads, as well as other electronics leads described herein, or other electronics leads that would be selected by the person skilled in the art.

The scope of the invention is defined by the appended claims.

## Claims

1. An implantable neural stimulation device comprising:
a body containing stimulation electronics and a battery to provide stimulation energy;
a first header coupled to the body of the device and containing a charge coil to charge the battery and a connector to connect a stimulation lead to the stimulation electronics to deliver the stimulation energy from the battery under control by the stimulation electronics; and
a second header coupled to the body and containing a communication antenna to provide communication between the stimulation electronics and an external communication device.

2. The device of claim 1, further comprising:
a first set of electronics leads to provide a link between the stimulation electronics and the first header, and a second set of electronics leads to provide a link between the stimulation electronics and the second header.

3. The device of claim 2, wherein the second set of electronics leads are located between the stimulation electronics and communication electronics.

4. The device of claim 2 or 3, wherein at least one of the first set of electronics leads and/or the second set of electronics leads are flexible leads, and the flexible leads are configured to collapse when the first header and/or the second header respectively are moved closer to the body.

5. The device of any one of claims 2 to 4, wherein the first set of electronics leads and/or the second set of electronics leads are coupled to the first header and second header respectively via a first feedthrough and/or a second feedthrough associated with the first header and the second header respectively, wherein the communications electronics are located between the second set of electronics leads and the second feedthrough.

6. The device of any one of claims 3 to 5, wherein the communication electronics are located proximal to the second header.

7. The implantable neural stimulation device of any of the preceding claims, wherein the body further contains:
communications electronics in communication with the stimulation electronics;
one or more support elements;
a second set of electronics leads to provide a link between the communications electronics and the communication antenna in the second header;
wherein the one or more support elements are configured to isolate the second set of electronics leads from a surface of the body.

8. The device of claim 7, wherein the one or more support elements are configured to guide the second set of electronics leads to collapse in a consistent specified configuration when the second header is coupled to the body.

9. The device of any one of the preceding claims, wherein the body further contains:
a printed circuit board;
a temperature sensor connected to the printed circuit board; and
a rectifier connected to the printed circuit board.

10. The implantable neural stimulation device of any one of the preceding claims, wherein:
the body comprises a first portion and a second portion; and
the first header is coupled to an end of the first portion of the body; and
the second header is coupled to another end of the first portion of the body.

11. The device of claim 10, wherein the first portion of the body and second portion of the body are welded together to form the body.

12. A method of manufacturing an implantable neural stimulation device, the device comprising a body with at least a first portion and a second portion to contain stimulation electronics and battery, a first header containing a charge coil, a second header containing a communications antenna, and at least one connector in the first and/or second header to connect a stimulation lead to stimulation electronics to deliver stimulation energy from the battery under the control of stimulation electronics, the method comprising:
forming a first portion with at least one lid, wherein the lid has feedthroughs to couple with components of respective first and second headers;
coupling the feedthroughs with at least the stimulation electronics and the battery;
attaching the first portion of the body to the second portion of the body to seal the stimulation electronics and battery inside the body; and
coupling respective first and second headers to the body, wherein components of the headers are in communication with components in the body via the feedthroughs.

13. The method of claim 12, wherein attaching the first portion of the body and the second portion of the body comprises welding the first portion of the body to the second portion of the body.

14. The method of claim 12 or 13 further comprising;
- locating a jig at, or relative to, the first portion to guide one or more flexible leads;
- guiding one or more flexible leads between the feedthroughs and at least the stimulation electronics and battery, wherein the one or more flexible leads are guided by the jig to collapse in a consistent, specified configuration; and
wherein before the step of attaching the first portion of the body to the second portion of the body, the method further comprises:
- removing the jig from the first portion.

15. The device of any one of claims 1 to 11, wherein the stimulation electronics are provided at a printed circuit board contained in the body, wherein the printed circuit board has at least one notch for at least one set of electronics leads, that links the printed circuit board to a first or second header, to collapse in the notch.

## Patentansprüche

1. Implantierbares Nervenstimulationsgerät, umfassend:
ein Gehäuse, das Stimulationselektronik und eine Batterie zur Bereitstellung von Stimulationsenergie enthält;
ein erstes Kopfteil, das mit dem Gehäuse des Geräts verbunden ist und eine Ladespule zum Aufladen der Batterie und einen Verbinder zum Verbinden einer Stimulationsleitung mit der Stimulationselektronik zum Zuführen der Stimulationsenergie von der Batterie unter Steuerung durch die Stimulationselektronik enthält; und
ein zweites Kopfteil, das mit dem Gehäuse verbunden ist und eine Kommunikationsantenne für die Kommunikation zwischen der Stimulationselektronik und einem externen Kommunikationsgerät enthält.

2. Gerät nach Anspruch 1, ferner umfassend:
einen ersten Satz Elektronikleitungen, um eine Verbindung zwischen der Stimulationselektronik und dem ersten Kopfteil bereitzustellen, und einen zweiten Satz Elektronikleitungen, um eine Verbindung zwischen der Stimulationselektronik und dem zweiten Kopfteil bereitzustellen.

3. Gerät nach Anspruch 2, wobei der zweite Satz Elektronikleitungen sich zwischen der Stimulationselektronik und der Kommunikationselektronik befindet.

4. Gerät nach Anspruch 2 oder 3, wobei die Leitungen von wenigstens einem der ersten und/oder zweiten Elektronikleitungssätze flexible Leitungen sind und die flexiblen Leitungen so konfiguriert sind, dass sie sich falten, wenn das erste Kopfteil bzw. das zweite Kopfteil näher an das Gehäuse herangeführt werden.

5. Gerät nach einem der Ansprüche 2 bis 4, wobei der erste Satz Elektronikleitungen und/oder der zweite Satz Elektronikleitungen mit dem ersten Kopfteil bzw. dem zweiten Kopfteil über eine erste Durchführung und/oder eine zweite Durchführung, welche zum ersten Kopfteil bzw. zum zweiten Kopfteil gehören, verbunden sind, wobei sich die Kommunikationselektronik zwischen dem zweiten Satz Elektronikleitungen und der zweiten Durchführung befindet.

6. Gerät nach einem der Ansprüche 3 bis 5, wobei die Kommunikationselektronik sich in der Nähe des zweiten Kopfteils befindet.

7. Implantierbares Nervenstimulationsgerät nach einem der vorhergehenden Ansprüche, wobei das Gehäuse ferner enthält:
Kommunikationselektronik in Kommunikation mit der Stimulationselektronik;
ein oder mehrere Trägerelemente;
einen zweiten Satz Elektronikleitungen zur Bereitstellung einer Verbindung zwischen der Kommunikationselektronik und der Kommunikationsantenne im zweiten Kopfteil;
wobei die ein oder mehreren Trägerelemente so konfiguriert sind, dass sie den zweiten Satz Elektronikleitungen von einer Oberfläche des Gehäuses isolieren.

8. Gerät nach Anspruch 7, wobei die ein oder mehreren Trägerelemente so konfiguriert sind, dass sie den zweiten Satz Elektronikleitungen so führen, dass diese in einer konsistenten festgelegten Konfiguration zusammenfalten, wenn das zweite Kopfteil mit dem Gehäuse verbunden wird.

9. Gerät nach einem der vorhergehenden Ansprüche, wobei das Gehäuse ferner enthält:
eine Leiterplatte;
einen Temperaturfühler, der mit der Leiterplatte verbunden ist; und
einen mit der Leiterplatte verbundenen Gleichrichter.

10. Implantierbares Nervenstimulationsgerät nach einem der vorhergehenden Ansprüche, wobei:
das Gehäuse einen ersten Teil und einen zweiten Teil umfasst; und
das erste Kopfteil mit einem Ende des ersten Teils des Gehäuses verbunden ist; und
das zweite Kopfteil mit einem anderen Ende des ersten Teils des Gehäuses verbunden ist.

11. Gerät nach Anspruch 10, wobei der erste Teil des Gehäuses und der zweite Teil des Gehäuses zur Bildung des Gehäuses miteinander verschweißt sind.

12. Verfahren zur Herstellung eines implantierbaren Nervenstimulationsgerät, wobei das Gerät ein Gehäuse mit wenigstens einem ersten Teil und einem zweiten Teil zur Aufnahme von Stimulationselektronik und Batterie, ein erstes Kopfteil, das eine Ladespule enthält, ein zweites Kopfteil, das eine Kommunikationsantenne enthält, und wenigstens einen Verbinder im ersten und/oder zweiten Kopfteil zur Verbindung einer Stimulationsleitung mit der Stimulationselektronik zum Zuführen von Stimulationsenergie von der Batterie unter Steuerung durch die Stimulationselektronik umfasst, wobei das Verfahren umfasst:
Bilden eines ersten Teils mit wenigstens einem Deckel, wobei der Deckel Durchführungen besitzt zur Verbindung mit Komponenten des ersten und zweiten Kopfteils;
Verbinden der Durchführungen mit wenigstens der Stimulationselektronik und der Batterie;
Befestigen des ersten Teils des Gehäuses am zweiten Teil des Gehäuses, um die Stimulationselektronik und die Batterie im inneren des Gehäuses einzuschließen; und
Verbinden des ersten und des zweiten Kopfteils mit dem Gehäuse, wobei Komponenten der Kopfteile über die Durchführungen mit Komponenten im Gehäuse in Verbindung stehen.

13. Verfahren nach Anspruch 12, wobei das Befestigen des ersten Teils des Gehäuses am zweiten Teil des Gehäuses das Verschweißen des ersten Teils des Gehäuse mit dem zweiten Teil des Gehäuses umfasst.

14. Verfahren nach Anspruch 12 oder 13, ferner umfassend:
- Anbringen einer Vorrichtung am oder relativ zum ersten Teil, um eine oder mehrere flexible Leitungen zu führen;
- Führen von einer oder mehreren flexiblen Leitungen zwischen den Durchführungen und wenigstens der Stimulationselektronik und der Batterie, wobei die ein oder mehreren flexiblen Leitungen so von der Vorrichtung geführt werden, dass sie in einer konsistenten festgelegten Konfiguration zusammenfalten; und
wobei vor dem Schritt des Befestigens des ersten Teils des Gehäuses am zweiten Teil des Gehäuses, das Verfahren ferner umfasst:
- Entfernen der Vorrichtung vom ersten Teil.

15. Gerät nach einem der Ansprüche 1 bis 11, wobei die Stimulationselektronik an einer im Gehäuse enthaltenen Leiterplatte bereitgestellt wird, wobei die Leiterplatte wenigstens eine Aussparung für wenigstens einen Satz Elektronikleitungen besitzt, der die Leiterplatte mit einem ersten oder zweiten Kopfteil verbindet, um in der Aussparung zusammenzufalten.

## Revendications

1. Dispositif de stimulation neuronale implantable comprenant :
un corps contenant une électronique de stimulation et une batterie pour fournir une énergie de stimulation ;
un premier collecteur couplé au corps du dispositif et contenant une bobine de charge pour charger la batterie et un connecteur pour connecter un fil de stimulation à l'électronique de stimulation afin de délivrer l'énergie de stimulation à partir de la batterie sous le contrôle de l'électronique de stimulation ; et
un second collecteur couplé au corps et contenant une antenne de communication pour assurer la communication entre l'électronique de stimulation et un dispositif de communication externe.

2. Dispositif selon la revendication 1, comprenant en outre :
un premier ensemble de fils électroniques pour assurer une liaison entre l'électronique de stimulation et le premier collecteur, et un second ensemble de fils électroniques pour assurer une liaison entre l'électronique de stimulation et le second collecteur.

3. Dispositif selon la revendication 2, dans lequel le second ensemble de fils électroniques est situé entre l'électronique de stimulation et l'électronique de communication.

4. Dispositif selon la revendication 2 ou 3, dans lequel au moins l'un du premier ensemble de fils électroniques et/ou du second ensemble de fils électroniques est des fils flexibles, et les fils flexibles sont configurés pour s'affaisser lorsque le premier collecteur et/ou le second collecteur respectivement sont rapprochés du corps.

5. Dispositif selon l'une quelconque des revendications 2 à 4, dans lequel le premier ensemble de fils électroniques et/ou le second ensemble de fils électroniques sont couplés au premier collecteur et au second collecteur respectivement via une première traversée et/ou une seconde traversée associées au premier collecteur et au second collecteur respectivement, dans lequel l'électronique de communication est située entre le second ensemble de fils électroniques et la seconde traversée.

6. Dispositif selon l'une quelconque des revendications 3 à 5, dans lequel l'électronique de communication est située proximale au second collecteur.

7. Dispositif de stimulation neuronale implantable selon l'une quelconque des revendications précédentes, dans lequel le corps contient en outre :
une électronique de communication en communication avec l'électronique de stimulation ;
un ou plusieurs éléments de support ;
un second ensemble de fils électroniques pour assurer une liaison entre l'électronique de communication et l'antenne de communication dans le second collecteur ;
dans lequel les un ou plusieurs éléments de support sont configurés pour isoler le second ensemble de fils électroniques d'une surface du corps.

8. Dispositif selon la revendication 7, dans lequel les un ou plusieurs éléments de support sont configurés pour guider le second ensemble de fils électroniques pour qu'ils s'affaissent dans une configuration spécifiée cohérente lorsque le second collecteur est couplé au corps.

9. Dispositif selon l'une quelconque des revendications précédentes, dans lequel le corps contient en outre :
une carte de circuit imprimé ;
un capteur de température connecté à la carte de circuit imprimé ; et
un redresseur connecté à la carte de circuit imprimé.

10. Dispositif de stimulation neuronale implantable selon l'une quelconque des revendications précédentes, dans lequel :
le corps comprend une première portion et une seconde portion ; et
le premier collecteur est couplé à une extrémité de la première portion du corps ; et
le second collecteur est couplé à une autre extrémité de la première portion du corps.

11. Dispositif selon la revendication 10, dans lequel la première portion du corps et la seconde portion du corps sont soudées ensemble pour former le corps.

12. Procédé de fabrication d'un dispositif de stimulation neuronale implantable, le dispositif comprenant un corps avec au moins une première portion et une seconde portion pour contenir une électronique de stimulation et une batterie, un premier collecteur contenant une bobine de charge, un second collecteur contenant une antenne de communication, et au moins un connecteur dans le premier et/ou le second collecteur pour connecter un fil de stimulation à l'électronique de stimulation afin de délivrer une énergie de stimulation à partir de la batterie sous le contrôle de l'électronique de stimulation, le procédé comprenant :
la formation d'une première portion avec au moins un couvercle, dans lequel le couvercle a des traversées pour se coupler avec des composants du premier et du second collecteur respectif ;
le couplage des traversées avec au moins l'électronique de stimulation et la batterie ;
l'attachement de la première portion du corps à la seconde portion du corps pour sceller l'électronique de stimulation et la batterie à l'intérieur du corps ; et
le couplage du premier et du second collecteur respectif au corps, dans lequel des composants des collecteurs sont en communication avec des composants du corps via les traversées.

13. Procédé selon la revendication 12, dans lequel l'attachement de la première portion du corps et de la seconde portion du corps comprend le soudage de la première portion du corps à la seconde portion du corps.

14. Procédé selon la revendication 12 ou 13 comprenant en outre :
- la localisation d'un gabarit au niveau de la première portion, ou par rapport à celle-ci, pour guider un ou plusieurs fils flexibles ;
- le guidage d'un ou de plusieurs fils flexibles entre les traversées et au moins l'électronique de stimulation et la batterie, dans lequel les un ou plusieurs fils flexibles sont guidés par le gabarit pour s'affaisser dans une configuration spécifiée, cohérente ; et
dans lequel, avant l'étape d'attachement de la première portion du corps à la seconde portion du corps, le procédé comprend en outre :
- le retrait du gabarit de la première portion.

15. Dispositif selon l'une quelconque des revendications 1 à 11, dans lequel l'électronique de stimulation est fournie à une carte de circuit imprimé contenue dans le corps, dans lequel la carte de circuit imprimé a au moins une encoche pour qu'au moins un ensemble de fils électroniques, qui relie la carte de circuit imprimé à un premier ou second collecteur, s'affaisse dans l'encoche.
